# EUROPÄISCHE PATENTSCHRIFT

(11) **EP 0 445 753 B1**
(45) Veröffentlichungstag und Bekanntmachung des Hinweises auf die Patenterteilung: **30.08.1995**
(21) Anmeldenummer: 91103358.7
(22) Anmeldetag: 06.03.1991
(51) Int. Cl.: B29D 31/00, F16C 13/00

(54) **Walzen mit Kunststoffbezug und Verfahren zur Herstellung derselben**
Rollers with plastic coating and process for their manufacture
Rouleaux avec revêtement en plastique et procédé pour leur fabrication

(30) Priorität: 07.03.1990 DE 4007141
(43) Veröffentlichungstag der Anmeldung: 11.09.1991
(73) Patentinhaber: Felix Böttcher GmbH & Co. KG, D-50933 Köln (DE)
(72) Erfinder: Weinert, Johann, Dr., W-5014 Kerpen (DE); Dau, Gerhard, Dipl.-Ing., Chemie, W-5100 Aachen (DE)
(74) Vertreter: Werner, Hans-Karsten, Dr.Dipl.-Chem.

(56) Entgegenhaltungen:
- EP-A- 0 113 575
- EP-A- 0 129 799
- EP-A- 0 232 540
- EP-A- 0 321 561
- DE-A- 2 328 738
- DE-A- 2 440 953
- GB-A- 1 306 458
- US-A- 4 466 164

## Beschreibung

Die Erfindung betrifft Walzen mit Kunststoffbezug und Verfahren zur Herstellung derselben gemäß den Merkmalen der Ansprüche 1 und 2.

Walzen mit Kunststoffbezug werden zu den verschiedensten Zwecken eingesetzt und nach den verschiedensten Verfahren hergestellt. Typische Kunststoffbezüge bestehen aus Gummi, Polyamiden, Polyurethanen, Polyestern, Polyesteramiden, Epoxidharzen, Silikonharzen, Phenolharzen, Polyacetatharzen, Polyamidharzen, Melaminharzen, etc. Je nach Verwendungszweck und jeweiligen Anforderungen werden diese Bezüge in bekannter Weise durch anorganische Füllstoffe, Flexibilisatoren, Impact-Modifier und sonstige Zusätze bezüglich der Härte, Abriebfestigkeit, Schlagbiegefestigkeit, Temperaturbeständigkeit sowie Beständigkeit gegen Lösungsmittel und Chemikalien modifiziert. Die Herstellung erfolgt im allgemeinen dadurch, daß auf den gegebenenfalls mit einer Haftschicht versehenen Walzenkern der Kunststoffbezug aufgegossen, aufextrudiert oder aufgewickelt wird. Die Aushärtung erfolgt bei Thermoplasten durch Abkühlen und bei den übrigen Kunststoffen durch anschließende Quervernetzung.

Ein Nachteil der meisten bisher verwendeten Kunststoffbezüge ist die realtiv geringe Temperaturbeständigkeit. So haben zum Beispiel Bezüge aus Silikon bzw. Fluorkautschuk zwar eine hohe Temperaturbeständigkeit, besitzen aber nicht die für spezielle Einsatzzwecke notwendige Härte. Die EP-A-0 321 561 beschreibt zwar Materialien, die gute Hitze- und Druckbeständigkeit aufweisen; es ist aber nicht möglich, das Problem der lokalen Überhitzung beim Betriebschnellrotierender Walzen zu lösen. Der Trend zu schnellerer Produktion und damit höheren Tourenzahlen erfordert aber zunehmend höhere Beständigkeit auch gegen hohe Temperaturen sowie gegen rasch wechselnde Temperaturen und gegen kurzfristige lokale Überhitzungen.

Die Erfindung hat sich somit die Aufgabe gestellt, Walzen mit derartigen hochtemperaturbeständigen Kunststoffbezügen zu entwickeln, die obendrein bezüglich Härte, Abriebfestigkeit, Schlagbiegefestigkeit, Beständigkeit gegen Lösungsmittel und Chemikalien sowie der sonstigen geforderten physikalischen Eigenschaften variabel eingestellt werden können.

Es wurde jetzt festgestellt, daß diese Aufgabe gelöst werden kann durch Bezüge aus Poly-2-Oxazolidinonen. Die Poly-2-Oxazolidinone können dabei im Gemisch mit gewissen Mengen an Isocyanuraten vorliegen. Sie können weiterhin durch Zumischung oder Einbau sonstiger Komponenten modifiziert werden.

Poly-2-Oxazolidinone entstehen beispielsweise bei der katalysierten Umsetzung von Diepoxiden mit Diisocyanaten bei Temperaturen über 120°C. Da unter diesen Bedingungen Isocyanate auch mit sich selbst zu Isocyanuraten reagieren, enthalten die so hergestellten Kunststoffbezüge im allgemeinen mehr oder weniger große Mengen an Isocyanuraten. Es ist jedoch durch Variation der Verfahrensbedingungen, Auswahl der Katalysatoren und sonstige modifizierende Zusätze möglich, die Bildung von Isocyanuraten zu unterdrücken oder zu verstärken und dadurch bereits die Eigenschaften des fertigen Kunststoffbezuges zu modifizieren. Weiterhin können selbstverständlich nicht nur Diepoxide und Diisocyanate, sondern auch andere mehrfunktionelle Epoxide und/oder Isocyanate verwendet werden.

Die Verknüpfung von Epoxiden mit Isocyanaten unter Bildung von Oxazolidinon-Strukturen ist an sich bekannt, jedoch wird diese Verknüpfung bisher technisch kaum im größeren Umfang genützt; vergl. Becker/Braun, Kunststoff Handbuch 10, "Duroplaste", Seite 111. Bekannt geworden sind bisher vor allem oligomere Oxazolidinon enthaltende Polyepoxide aus Bisepoxiden und Diisocyanaten gemäß EP-OS-0 296 450. Diese Oligomeren sind vor allem mit anderen flüssigen oder festen Epoxidharzen oder Reaktiv-Verdünnern abgemischt und mit Hilfe der üblichen Härter, wie Polyaminen, Säureanhydriden oder Polyisocyanaten, gehärtet worden. Die so erhaltenen Produkte zeigen erhöhte Reißfestigkeit, Reißdehnung, Schlagzähigkeit, Kugeldruckhärte und Wärmeformbeständigkeit. Sie sind aber bisher nicht dazu verwendet worden, mit nur relativ geringen Mengen eines Katalysators mit sich selbst zu reagieren und ausgehärtet zu werden. Dies ist insbesondere darauf zurückzuführen, daß dabei sehr harte und spröde und deshalb letztlich unbrauchbare Produkte entstehen. Es wurde jetzt gefunden, daß es jedoch möglich ist, derartige oligomere Präpolymere mit Isocyanurat-Gruppen mit Hilfe von Katalysatoren und relativ geringen Mengen von Flexibilisatoren auszuhärten und dabei Produkte zu erhalten, die ausgezeichnet geeignet sind, als harte Kunststoffbezüge für Walzen eingesetzt zu werden. Es wurde weiterhin festgestellt, daß es ohne weiteres möglich ist, die für die Herstellung verwendeten Diepoxide und Diisocyanate mit einem Katalysator und einem Flexibilisator umzusetzen und dabei Produkte zu erhalten, die je nach Reaktionsbedingungen einen wesentlich geringeren Gehalt an Isocyanurat-Gruppen enthalten.

Es wurde weiterhin gefunden, daß durch Variation der eingesetzten mehrfunktionellen Epoxide einerseits und der mehrfunktionellen Isocyanate andererseits die Eigenschaften des Kunststoffes erheblich variiert werden können und damit den jeweils geforderten Eigenschaften an einen Kunststoffbezug für Walzen angepaßt werden können. Es ist auch möglich, diese Kunststoffe durch anorganische Füllstoffe zu variieren und zu modifizieren.

Allen derartigen erfindungsgemäßen Kunststoffbezügen ist jedoch gemeinsam, daß sie im Vergleich zu den bisher verwendeten Kunststoffen eine wesentlich höhere Temperaturbeständigkeit und Beständigkeit gegen Temperaturschwankungen aufweisen und trotzdem flexibel genug sind.

Dies ist insbesondere wohl darauf zurückzuführen, daß sowohl die Oxazolidinon-Struktur, als auch die Isocyanurat-Struktur sehr unempfindlich sind gegen Temperaturbelastung. Da reine Poly-2-Oxazolidinone und auch solche im Gemisch mit Isocyanuraten, insbesondere wenn sie aus Diepoxiden auf Basis von Bisphenol-A und Diisocyanaten auf Basis von Diphenylmethan hergestellt wurden, sehr spröde sind, wurde diese Gruppe von Kunststoffen bisher wenig beachtet. Es war daher nicht vorherzusehen, daß diese Kunststoffe bereits durch geringfügige Modifikation und insbesondere den Einsatz von Flexibilisatoren zu ausgezeichneten Kunststoffbezügen für Walzen weiterentwickelt werden könnten.

Als Bisepoxide können erfindungsgemäß jedoch nicht nur solche auf Basis von Bisphenol-A eingesetzt werden, sondern auch andere handelsübliche Bisepoxide, die als Epoxidkomponenten in Kunststoffen zum Einsatz gekommen sind. Eine Zusammenstellung derartiger Bisepoxide findet sich in Becker/Braun, Kunststoff Handbuch 10,"Duroplaste", Seiten 115 bis 118. Weiterhin können auch Komponenten mit drei oder mehr Epoxidgruppen verwendet werden.

Als Diisocyanat-Komponente kommen ebenfalls prinzipiell alle technisch zugänglichen Diisocyanate in Frage. Weiterhin kommen auch Substanzen mit drei oder mehr Isocyanatgruppen in Frage.

Als Katalysatoren für die Addition von Epoxiden mit Isocyanaten kommen die verschiedensten Produktgruppen in Frage. Geeignet sind beispielsweise tertiäre Basen, wie Pyridin, Tribenzylamin, N-Methyldibenzylamin, aber auch Lewis-Säuren, wie Zinkchlorid, Eisenchlorid, Bortrifluorid, Alkali- und Erdalkalialkoholate, wie Calcium- und Magnesiumethylat sowie Aluminium-Isopropylat. Weitere Katalysatoren sind Lithiumchlorid, Tetramethylammoniumjodid sowie Phosphoniumsalze.

Als Flexibilisatoren kommen insbesondere Substanzen in Frage, deren funktionellen Gruppen im Molekül im weiten Abstand über vornehmlich aliphatische Strukturelemente miteinander verbunden sind. Typische Flexibilisatoren sind Polypropylenglykol-Diglycidylether, Oligoester, Oligoetherester mit endständigen Carboxylgruppen, Amingruppen oder Hydroxylgruppen, die zumindest bei höheren Temperaturen mit den noch vorhandenen Epoxidgruppen oder Isocyanatgruppen reagieren können und dabei zu einer flexibilisierenden Vernetzung führen. Gute flexibilisierende Eigenschaften haben daher auch flexibilisierte Epoxide, Polybutadien, Polyisopren sowie Polybutadiene mit endständigen Epoxid- oder Hydroxylgruppen.

Als anorganische Füllstoffe kommen prinzipiell alle bekannten anorganischen Füllstoffe in Frage, die auch jetzt schon als Füllstoffe für Kunststoffe und insbesondere Kunststoffbezüge von Walzen verwendet werden. Beispielsweise sind Aluminiumoxide, Siliciumdioxide, Siliciumcarbide und Granitmehl gut geeignet, wenn harte, griffige und verschleißarme Überzüge gewünscht werden.

Die Herstellung der Kunststoffbezüge auf den Walzen erfolgt ebenfalls in an sich bekannter und üblicher Weise. Dazu wird der Walzenkern üblicherweise entfettet und gesandstrahlt. Häufig empfiehlt es sich, eine Haftschicht als Primer aufzutragen, beispielsweise ein Phenolharz. Für harte Walzen empfiehlt es sich weiterhin als Spannungsausgleich zwischen Kern und Kunststoffbezug eine Polyurethanschicht aufzutragen, auf der dann der erfindungsgemäße Kunststoffbezug aus Poly-2-Oxazolidinon aufgebracht wird. Dies kann bei Temperaturen bis zu 100°C erfolgen. Die Aushärtung erfolgt dann bei Temperaturen über 120°C. Vorzugsweise erfolgt das Härten bei Temperaturen zwischen 130 und 180°C. Um blasenfreie Kunststoffbezüge zu erhalten, empfiehlt es sich, die noch flüssigen Komponenten unter Vakuum zu entgasen und zu homogenisieren. Sofern ein höherer Anteil an Isocyanurat-Strukturen gewünscht wird, kann beispielsweise die Diisocyanat-Komponente zuvor mit Katalysatoren, wie Alkali- und Erdalkalialkoholaten erhitzt werden, bei der die Isocyanurat-Struktur entsteht. Eine weitere Möglichkeit zur Erhöhung der Isocyanurat-Struktur ist der Einsatz von oligomeren Präpolymeren, wie sie beispielsweise in der EP-OS-0 296 450 beschrieben sind.

Gegenstand der vorliegenden Erfindung sind somit nicht die Walzen mit Kunststoffbezug (Anspruch 1) im allgemeinen und das Verfahren (Anspruch 2) zu ihrer Herstellung, sondern die Verwendung von Poly-2-Oxazolidinonen gegebenenfalls im Gemisch mit Isocyanuraten sowie Flexibilisatoren, Füllstoffen und sonstigen Zusätzen als Werkstoff für Walzenbezüge. Ein weiterer Gegenstand der Erfindung ist die Verwendung von oligomeren Oxazolidinon-Gruppen enthaltenden Präpolymeren aus Diepoxiden und Diisocyanaten zur Herstellung von Walzenbezügen.

In den nachfolgenden Beispielen sind einige Ausführungsbeispiele erfindungsgemäßer Walzen und das Verfahren zu ihrer Herstellung beschrieben. Die Eigenschaften sind jedoch dem jeweiligen Anwendungs- und Verwendungszweck entsprechend ohne weiteres im weiten Umfang variierbar.

### BEISPIEL 1

20 Gew.-Teile Diepoxid auf Basis von Bisphenol-A (Lekutherm X 18) werden mit 80 Gew.-Teilen eines Diisocyanats (Gemisch aus 4,4- und 4,2-Diisocyanatodiphenylmethan (MDI), Baymidur VP 5002) vermischt und mit 5 Gew.-Teilen N-Methyl-Dibenzylamin (als Katalysator) und 10 - 30 Gew.-Teilen Polybutadien (R 20 LM mit endständigen Hydroxylgruppen) vermischt. Dem Gemisch werden 160 bis 220 Gew.-Teile Siliciumcarbid zugegeben. Das bei 80°C entgaste und homogenisierte Gemisch wird auf einen Walzenkern mit Haftschicht und ca. 1 mm starker Polyurethanschicht vorbeschichteten Walzenkern gegossen und bei 130°C gehärtet. Der so erhaltene Kunststoffbezug weist alle geforderten physikalischen Eigenschaften auf und ist obendrein hochtemperaturbeständig.

### BEISPIEL 2

In analoger Weise wie im Beispiel 1 beschrieben werden 100 Gew.-Teile eines Präpolymeren eingesetzt, das aus 20 Gew.-Teilen des gleichen Diepoxids und des gleichen Diisocyanats hergestellt wurde, wobei das Präpolymere noch ca. 20 % freie Isocyanatgruppen und darüber hinaus im erheblichen Umfang Isocyanuratgruppen enthält (Blendur I-VP KU 3-4516 der Bayer AG).

### BEISPIEL 3

In analoger Weise wie im Beispiel 2 beschrieben werden 100 Gew.-Teilen des gleichen Präpolymeren (Blendur KU 3-4516) vermischt mit 15 Gew.-Teilen Diepoxid auf Basis Bisphenol-A (Epikote 828), 15 Gew.-Teilen Hexamethylendiisocyanat mit Isocynanatgruppen (Desmodur N 3300) und 10 Gew.-Teilen N-Methyl-Dibenzylamin als Katalysator. Weiterhin werden 160 bis 200 Gew.-Teile Siliciumcarbid eingemischt. Das bei 80°C entgaste und homogenisierte Gemisch wird auf einen Walzenkern mit Haftschicht und ca. 1 mm starker Polyurethanschicht vorbeschichteten Walzenkern gegossen und bei 130°C gehärtet. Der so erhaltene Kunststoffbezug weist alle geforderten physikalischen Eigenschaften auf und ist obendrein hochtemperaturbeständig.

### BEISPIEL 4

In analoger Weise wie im Beispiel 3 beschrieben werden 100 Gew.-Teile des Präpolymeren (Blendur KU3-4516) mit 12. Gew.-Teilen Butan-1.4-diol-diglycidylether (Araldit DY 026 SP), 9 Gew.-Teilen Hexamethylendiisocyanat (Desmodur N 3200), 10 Gew.-Teilen N-Methyl-Dibenzylamin und 160 bis 220 Gew.-Teilen Siliciumcarbid vermischt und zu einem Walzenbezug verarbeitet. Der so erhaltene Kunststoffbezug weist alle geforderten physikalischen Eigenschaften auf und ist obendrein hochtemperaturbeständig.

### BEISPIEL 5

In analoger Weise wie im Beispiel 1 beschrieben werden 100 Gew.-Teile Butan-1.4-diol-diglycidylether (Araldit DY 026 SP) mit 80 Gew.-Teilen Hexamethylendiisocyanat (Desmodur N 3200), 18. Gew.-Teilen N-Methyl-Dibenzylamin und bis zu 220 Gew.-Teilen Füllstoffe vermischt und zu einem Walzenüberzug verarbeitet. Diese Schicht ist relativ weich und weist eine relativ niedrige Härte auf (ca. 50 Shore A). Es ist somit möglich, diese Rezeptur mit härteren Rezepturen zu verschneiden, um die gewünschte Härte einzustellen.

## Patentansprüche

1. Walzen mit Kunststoffbezug, dadurch gekennzeichnet, daß der Bezug aus Poly-2-Oxazolidinonen im Gemisch mit Isocyanuraten besteht, durch Flexibilisatoren modifiziert ist und anorganische Füllstoffe enthält.

2. Verfahren zur Herstellung von Walzen mit Kunststoffbezug, dadurch gekennzeichnet, daß auf den gegebenenfalls zuvor mit einer Haftschicht versehenen Walzenkernen ein Gemisch aus mehrfunktionellen Epoxiden, mehrfunktionellen Isocyanaten und einem Katalysator, unter Zusatz von Flexibilisatoren und anorganischen Füllstoffen aufgetragen und durch Erwärmen auf Temperaturen über 120°C ausgehärtet wird.

3. Verfahren gemäß Anspruch 2, dadurch gekennzeichnet, daß die Komponenten überwiegend oder ganz in Form von oligomeren Präpolymeren aus Diepoxiden und Diisocyanaten eingesetzt werden.

4. Verwendung von Poly-2-Oxazolidinonen im Gemisch mit Isocyanuraten sowie Flexibilisatoren, Füllstoffen und sonstigen Zusätzen als Werkstoff für Walzenbezüge.

5. Verwendung gemäß Anspruch 4, dadurch gekennzeichnet, daß oligomere Oxazolidinongruppen enthaltende Präpolymere aus Diepoxiden und Diisocyanaten verwendet werden.

## Claims

1. Rolls having a synthetic coating, characterized in that the coating consists of poly-2-oxazolidinones in a mixture with isocyanurates, is modified by flexibilizers and contains inorganic fillers.

2. Process for manufacturing rolls having a synthetic coating, characterized in that onto the roll shells being optionally provided with an adhesive layer in advance, a mixture of multifunctional epoxides, multifunctional isocyanates, and a catalyst, with addition of flexibilizers and inorganic fillers is applied, and cured by heating to temperatures above 120°C.

3. Process according to claim 2, characterized in that the components are used predominantly or entirely in the form of oligomeric pre-polymers made of diepoxides and diisocyanates.

4. Use of poly-2-oxazolidinones, admixed with isocyanurates as well as flexibilizers, fillers, and other additives, as a material for roll covers.

5. Use accordimg to claim 4, characterized in that oligomeric pre-polymers containing oxazolidinone moieties, being made of diepoxides and diisocyanates, are used.

## Revendications

1. Cylindres avec un revêtement en matière plastique, caractérisés en ce que le revêtement est formé de poly-2-oxazolidinones mélangées avec des isocyanurates, est modifié avec des agents d'assouplissement et contient des charges minérales.

2. Procédé de fabrication de cylindres avec un revêtement en matière plastique, caractérisé en ce qu'on applique, sur les cylindres, éventuellement préalablement pourvus d'une couche adhésive, un mélange d'époxydes polyfonctionnels, d'isocyanates polyfonctionnels et d'un catalyseur, avec addition d'agents d'assouplissement et de charges minérales, et on le durcit en le chauffant à des températures supérieures à 120°C.

3. Procédé selon la revendication 2, caractérisé en ce qu'on introduit les composants essentiellement ou totalement sous forme de prépolymères oligomères de diépoxydes et de diisocyanates.

4. Utilisation de poly-2-oxazolidinones mélangées avec des isocyanurates, ainsi que des agents d'assouplissement, des charges minérales et d'autres additifs, comme matériau pour des revêtement de cylindres.

5. Utilisation selon la revendication 4, caractérisée en ce qu'on utilise des prépolymères oligomères de diépoxydes et de diisocyanates, qui contiennent des groupes d'oxazolidinones.
